# EUROPEAN PATENT SPECIFICATION

(11) **EP 1 335 678 B1**
(45) Date of publication and mention of the grant of the patent: **24.09.2008**
(21) Application number: 01926750.9
(22) Date of filing: 06.04.2001
(51) Int. Cl.: A61C 17/34, A46B 13/04, A46B 11/00

(54) **ORAL TREATMENT SYSTEMS**
ORALE BEHANDLUNGSVORRICHTUNG
SYSTEMES DE TRAITEMENT BUCCAL

(30) Priority: 22.11.2000 US 721431
(43) Date of publication of application: 20.08.2003
(73) Proprietor: THE PROCTER & GAMBLE COMPANY, Cincinnati, Ohio 45202 (US)
(72) Inventor: HEALEY, Patrick John, West Chester, OH 45069 (US); VILTRO, Louis John, Hamilton, OH 45013 (US); WRIGHT, Scott Matthew, Cincinnati, OH 45226 (US); DE AMICIS, Giovanni Battista, Cincinnati, OH 45230 (US); LEIGH, Michael James, Frimley, Surrey GU16 5TB (GB); WHITE, Christopher David, Richmond, Surrey TW9 3EB (GB)
(74) Representative: Clemo, Nicholas Graham
(86) International application number: PCT/US2001/011453
(87) International publication number: WO 2002/041801

(56) References cited:
- WO-A-92/10146
- DE-A- 19 717 868
- US-A- 4 687 663
- US-A- 5 321 866

## Description

### Field of the Invention

The present invention relates to an oral treatment system comprising two or more oral treatment compositions held within a single, hand-held, electrically powered housing. In particular, the invention relates to an electric toothbrush capable of sequentially delivering two or more oral treatment compositions integrated into the brush handle. The system provides improved oral treatment regimens.

### Backgound Of The Invention

It still remains the case that most regular oral treatments are provided via the application of a single dentifrice product during tooth brushing. Such application generally relies on a user dispensing a product onto a brush head from a tube of such product. Very recently, electric toothbrushes, historically rather expensive, have become much more affordable and therefore more widespread. The basic process however, of squeezing toothpaste onto the brush head remains the same.

Whether using a manual or powered toothbrush in this way, a problem arises with placing a dentifrice on the bristles, in that there is a lack of consistency of delivery of the dentifrice. For example, the concentration of dentifrice on the bristles is at a maximum just after application of a dentifrice to the bristles, prior to brushing. Commencement of brushing quickly results in a decrease of the concentration of dentifrice on the bristles, and a possible reduction in the advantage provided by the dentifrice.

While it is possible to occasionally stop brushing, and reapply the dentifrice to the bristles, a more practical approach would be to provide the toothbrush with a reservoir from which the dentifrice is dispensed during brushing, either intermittently or continuously.

Such an idea is not new. For example, U.S. Patent No. 730,040, issued June 2, 1903 to McKinley et al., discloses a toothbrush having a receptacle for feeding a liquid dentifrice into the bristles of the brush. U.S. Patent No. 3,217,720, issued November 16, 1965 to Cyzer, discloses a toothbrush with a liquid dentifrice container. U.S. Patent 5,909,977, issued June 8, 1999 to Kuo, discloses a dentifrice dispensing toothbrush utilizing a refillable cartridge for storing dentifrice material and a compressible elastic button for pumping dentifrice material to the brush head. Further development of this idea includes the use of hollow bristles through which the dentifrice flows as disclosed in U.S. Patent No. 5,309,590, issued May 10,1994 to Giuliani et al.

A further problem with most oral treatment regimens is that they are limited to those treatments which can be provided within a single chemically stable composition, thus excluding treatment compositions which may be more efficacious but have the deficiency of being unstable on storage, or compositions whose components are best delivered sequentially.

Dual component dentifrice compositions are known in which a single product encompasses two distinct phases. Typical are those described in US patent no. 4,687,633 and in PCT applications WO 00/32159, WO 00/42981 and WO 99/55297. In such products, chemically incompatible components may be kept separated in distinct compartments of a flexible tube or other dispenser until dispensed onto a toothbrush, generally as two, side by side, ribbons. WO 00/62748 describes an oral care system in which two distinct products are provided, e.g. as a kit, which may be applied sequentially to the oral cavity. German publications DE 198 01 111 A1 and DE 297 21 297 U1 describe manual toothbrushes with two integrated cavities in which separate products may be stored for direct dispensing onto the brush head through a connecting channel. These brushes however do no permit a convenient substitution of one product for another since there is no straightforward mechanism for removal of residues of the first product when it is desired to substitute it with a second.

There remains a need therefore for an oral care system which can provide a variety of efficacious treatment regimens with the convenience expected by the modern day consumer. These and other needs will become apparent to those of skill in the art upon review of this specification.

### Summary Of The Invention

According to one embodiment of the present invention, there is provided an oral treatment system comprising a unitary housing, that can be comfortably gripped in a user's hand, the housing containing a cartridge comprising first and second reservoirs. The reservoirs, which are replaceably removable from the housing, respectively contain first and second, liquid, oral treatment compositions, the first and second compositions being different to each other. The system further comprises an applicator attached or attachable to the housing for applying the first and second oral treatment compositions to the teeth or oral soft tissues; an electrical power source to provide energy for pumping the compositions to the applicator and / or to supply energy to the applicator, and control means to allow the user to simultaneously or sequentially deliver the first and second compositions to the oral cavity. The system yet further comprises a set of instructions for the user.

According to a further aspect of the invention, there is provided a kit comprising a unitary cartridge adapted for use in the oral treatment system, the cartridge comprising first and second reservoirs respectively containing a first and second, liquid oral treatment compositions and a medium comprising a set of executable instructions for activating a program function of the oral treatment system.

### Detailed Description Of The Invention

The system of the present invention includes a cartridge for storing two or more liquid, oral treatment compositions. In a highly preferred embodiment the cartridge is replaceably removable from the housing, for example to allow the reservoirs to be refilled and the cartridge reinserted, for the cartridge to be replaced by one of substantially identical construction which may comprise further quantities of the same oral treatment compositions or may provide different compositions to supply a different oral treatment regimen. A suitable housing, cartridge and applicator assembly is described in EP 1,335,679. An applicator is attached or attachable to the housing through for applying the treatment compositions to the oral cavity. The applicator may be any suitable device for applying the compositions to teeth or oral soft tissues, and includes an application surface which may be a brush or a sponge. Preferably the applicator is a brush. Suitably, one or more channels run through the applicator to provide fluid communication between the reservoirs and the application surface.

Any suitable reservoir or cartridge may be utilized in the present invention. It should be understood that the reservoir or cartridge utilized may be fully or partially internal to the housing, or fully or partially external to the housing. Non-limiting examples of suitable reservoirs include positive displacement type reservoirs that are generally rigid-walled such as a cartridge, and also include pump-evacuated type reservoirs that are generally soft-walled such as sachets, bladders, and blisters.

An electrical power source is provided within the housing to provide energy for pumping the compositions to the applicator and / or to supply energy to the applicator, for example to drive a movable brush head. The power source can also be used to provide sonic energy to the compositions, as described in U.S. Patent No. 5,309,590, issued May 10, 1994 to Giuliani et al.

Control means, such as a switch or set of switches, optionally associated with a suitable microprocessor, allow the user to simultaneously or sequentially deliver the first and second compositions to the oral cavity. The use of a microprocessor is preferred since, by this means, precise, controlled dosage of treatment compositions can be achieved, or complex treatment regimens delivered. The microprocessor can be preprogrammed or programmable. Of course, to enhance oral care this microprocessor could be programmed to provide alarms or messages to a small display screen to remind the user to dispense certain compositions at certain times. For example, for two oral treatments where one is to be dispensed from the first reservoir every 4 hours, the other to be dispensed from the second reservoir every six hours, the microprocessor could be programmed to so dispense, with reminders/alarms to the user to use the brush at the appropriate times.

With or without a microprocessor, the ability to select simultaneous or sequential treatments from the two or more reservoirs allows for a wide variety of oral treatment regimens.

It is even envisioned that a given pre-loaded cartridge may further include its own executable instructions on a medium (for example a small chip, disk, microprocessor, or Radio Frequency ID tag to activate proprietary program functions) that is loaded into the housing to dispense material from the cartridge reservoirs as desired.

Necessarily, for an oral treatment system this complex a set of instructions needs to be provided for the user. Typically this would be done by the enclosure of a separate leaflet or small booklet enclosed with the system as part of a kit. A part of the instruction set could be integrated into the system via a microprocessor which communicates with the user via, for example, a LCD display screen, for example to give a simple instruction such as 'Now Select Switch B' or 'Battery replacement required'. The instructions may provide advice on the selection of the first and second compositions, according to the user's oral care needs. These instructions may relate solely to the use of the compositions present in the system on purchase, or may extend to advice on which auxiliary compositions to use, such as the selection of a replacement cartridge. The instructions may further provide advice on a treatment regimen to be applied with the system, according to the user's oral care needs, for example advice on what times of day to use the system and what treatments to select.

In yet a further embodiment of the invention, provision may be made for in situ refilling of the reservoirs contained within the housing from a separate refill cartridge, external to the housing, which is further provided with the system. This can be advantageous where the volumes of composition consumed during treatments are relatively large and it would be costly or wasteful to throw away an internal cartridge each time it was emptied. The external refill cartridge may form part of a base station that additionally performs the function of recharging a rechargeable battery within the housing.

### Treatment Compositions

A wide variety of treatment compositions can be provided for use with the system. Preferably, a first treatment composition includes a cleaning agent. Typically such a composition comprises from 0.1 to 40% of a cleaning agent selected from surfactants; abrasives and mixtures thereof. A wide variety of surfactants and abrasives is known in the art for oral care. Preferred surfactant levels are from 0.1% to 5%, more preferably from 0.5% to 5%. All percentages quoted herein are by weight of the respective treatment composition. Preferred abrasive levels are from 5% to 40%, more preferably from 5% to 25%. Suitable abrasives include calcium carbonate, precipitated silicas and insoluble phosphates.

Other preferred oral treatment compositions comprises from 0.01 to 20% of a conditioning agent selected from animal oils, mineral oils, vegetable oils and silicone oils, polymers which are substantive to the surfaces of the teeth or oral soft tissues; and mixtures thereof. A suitable vegetable oil is sunflower oil. Preferred conditioning agents are silicone oils. Suitable classes of silicone oils include, but are not limited to, dimethicones, dimethiconols, dimethicone copolyols and aminoalkylsilicones. More detail of these materials is provided in WO97/17939. Preferred aminoalkylsilicones comprise amodimethicones, such as OSI's Magnasoft® fluid. More preferred for use herein are alkyl- and alkoxy-dimethicone copolyols and mixtures thereof. Especially preferred are the dimethicone copolyols disclosed in WO96/33693. Such dimethicone copolyols have utility as antiplaque agents. In preferred embodiments, the dimethicone copolyol is selected from C₁₂ to C₂₀ alkyl dimethicone copolyols and mixtures thereof. Highly preferred is cetyl dimethicone copolyol marketed under the Trade Name Abil EM90.

Preferably at least one of the first and second treatment compositions comprises a fluoride ion source. Non-limiting examples of suitable fluoride ion sources include stannous fluoride, sodium fluoride, and sodium monofluorophosphate. The preferred fluoride ion source is sodium fluoride. Preferred compositions comprises a fluoride ion source providing from 100 to 3000 ppm of fluoride ion.

Other suitable oral treatment composition components include solvents, diluents, thickening agents, and the like to provide support for the treatment composition while dispensed on the applicator surface.

Yet further useful components include antimicrobials such as triclosan; anticalculus agents such as tetrasodium and tetrapotassium pyrophosphates and the polyphosphates disclosed in WO 98/22079; humectants, sweeteners and flavoring oils. All these components can be used at their art known levels.

The oral treatment compositions of the present invention will preferably have a viscosity amenable to pumping from the reservoirs to the applicator surface whilst avoiding being too thin so that the composition runs out of the applicator before use.

As used herein, the term "viscosity" refers to "dynamic viscosity" and is defined as the ratio of the shearing stress to the rate of deformation. A preferred viscosity for the first and second oral treatment compositions is fro about 10 to about 500 Pa.s when measured at a shear rate of 0.1 s⁻¹ at 25°C. These viscosities encompass both pourable liquids and gels which can be made to flow under a shear stress.

### Treatment Regimens

The system can deliver two compositions entirely simultaneously. This is advantageous where different chemical components are required simultaneously but cannot be stably stored together in one composition. General examples here would include an active ingredient and an activator for the ingredient. Specific examples here include the many peroxide whitening treatments known in the art, such as those disclosed in WO 98/31331.

In preferred embodiments the first and second treatment compositions are delivered sequentially to the teeth or oral soft tissues. The first and second treatment compositions can be delivered within the same treatment occasion, such as when both are both delivered within a period of five minutes. In these instances the timing of delivery of the two compositions may overlap provided that one treatment starts before or finishes after the other. Preferably the two treatments are cleanly separated so that there is no simultaneous delivery. A suitable treatment regimen here includes a first cleaning treatment followed immediately by a second conditioning treatment which may provide a protective coating to prevent plaque build up through the day.

Alternately there may be a substantial intervening rest period between the delivery of the first and second compositions. A suitable rest period could be at least six hours, for example when one composition is delivered in a morning treatment and another is delivered in an evening treatment. Usage instructions for this type of regimen may include means for reminding the user which composition is to be used. A suitable means may be a card based switch which the user resets after each usage. Alternately microprocessor control may alert the user via a visual display or audible indication (such as different tones) or may entirely automate the process. A suitable treatment regimen here may include a cleaning treatment in the morning, perhaps with only the first composition being delivered, succeeded by an evening treatment in which the cleaning composition is again delivered first but is then followed immediately by a second specialist remineralisation treatment for intensive overnight action.

### Examples

Formula 1A is a cleaning composition useful in the present invention.

| 1A | |
|---|---|
| Ingredient | % Wt/Wt |
| Sorbitol (70%), low reducing sugars | 60.0 |
| Glycerin | 10.0 |
| Xanthan gum | 0.5 |
| Sodium fluoride | 0.24 |
| Sodium saccharin | 0.3 |
| Flavour | 1.5 |
| Triclosan | 0.28 |
| Sodium lauryl sulphate solution (28%) | 8.0 |
| PEG-6 | 4.0 |
| Hydrated silica, amorphous | 10.0 |
| Water | to 100% |

Formula 1B is a moisturising composition useful for night time use.

| 1B | |
|---|---|
| Ingredient | % Wt/Wt |
| Glycerin | 30.0 |
| Xylitol | 20.0 |
| Xanthan gum | 0.5 |
| Sodium fluoride | 0.24 |
| Sodium benzoate | 0.1 |
| Titanium dioxide | 0.5 |
| Sodium saccharin | 0.3 |
| Flavour | 1.0 |
| Betaine | 4.0 |
| Sodium lauryl sulphate solution (28%) | 4.0 |
| Hydrated silica, amorphous | 10.0 |
| Water | to 100% |

Formulae 1A and 1B are provided in separate reservoirs within the system, which is fitted with an applicator brush, and the user is provided with instructions to actuate control switches in order to deliver Formula 1A during morning brushing and Formula 1B for night-time brushing.

Optionally, the system may be provided with an alternate cartridge containing Formula 1A in the first reservoir and a mineralising gel comprising a substantive fluoride treatment in the second reservoir so that Formula 1A is delivered during morning brushing and the fluoride treatment is delivered during night-time brushing. Sonic energy is delivered to the treatment compositions during each brushing occasion.

While the illustrative embodiments of the invention have been described with particularity, it will be understood that various other modifications will be apparent to and can be readily made by those skilled in the art without departing from the scope of the invention. In particular, whilst the examples imply a system which encompasses two treatment composition reservoirs within a cartridge, more complex cartridges which contain three, four or more reservoirs may be envisaged.

## Claims

1. An oral treatment system comprising:
(a) a unitary housing, that can be comfortably gripped in a user's hand, the housing containing a cartridge comprising:
(i) a first reservoir containing a first, liquid oral treatment composition;
(ii)a second reservoir containing a second, liquid oral treatment composition, which is different to the first oral treatment composition;
(b) an applicator attached or attachable to the housing for applying the first and second oral treatment compositions to the teeth or oral soft tissues;
(c) an electrical power source to provide energy for pumping the compositions to the applicator and / or to supply energy to the applicator;
(d) control means to allow the user to simultaneously or sequentially deliver the first and second compositions to the oral cavity; and
(e) a set of instructions for the user;
wherein the first and second reservoirs are replaceably removable from the housing.

2. A system of Claim 1 wherein an oral treatment composition comprises from 0.1 to 40% of a cleaning agent selected from surfactants; abrasives and mixtures thereof.

3. A system according to any preceding claim wherein an oral treatment composition comprises from 0.01 to 20% of a conditioning agent selected from animal oils, mineral oils, vegetable oils and silicone oils, polymers which are substantive to the surfaces of the teeth or oral soft tissues; and mixtures thereof.

4. A system according to any preceding claim wherein an oral treatment composition comprises a fluoride ion source providing from 100 to 3000 ppm of fluoride ion.

5. A system according to any preceding claim wherein the first and second oral treatment compositions have a viscosity of from 10 to 500 Pa.s when measured at a shear rate of 0.1 s⁻¹ at 25°C.

6. A system according to any preceding claim wherein the control means comprises a microprocessor.

7. A system according to Claim 6 wherein the microprocessor is programmable.

8. A kit comprising a unitary cartridge adapted for use in an oral treatment system according to any preceding claim, the cartridge comprising
(a) first and second reservoirs respectively containing a first and second, liquid oral treatment compositions; and
(b) a medium comprising a set of executable instructions for activating a program function of the oral treatment system.

9. A kit according to Claim9 wherein the medium is a RFID tag.

## Patentansprüche

1. Orales Behandlungssystem, umfassend:
(a) ein einheitliches Gehäuse, das bequem in der Hand eines Anwenders liegt, wobei das Gehäuse eine Kartusche umfasst, welche aufweist:
(i) ein erstes Reservoir, das eine erste, flüssige orale Behandlungszusammensetzung enthält;
(ii) ein zweites Reservoir, das eine zweite, flüssige orale Behandlungszusammensetzung enthält, die sich von der ersten oralen Behandlungszusammensetzung unterscheidet;
(b) einen Applikator, der am Gehäuse befestigt ist oder befestigt werden kann, um die erste und die zweite orale Behandlungszusammensetzung auf die Zähne oder die weichen oralen Gewebe aufzutragen;
(c) eine Quelle für elektrische Leistung, um Energie zum Pumpen der Zusammensetzungen zum Applikator und/oder zum Liefern von Energie zum Applikator bereitzustellen;
(d) Steuermittel, um dem Anwender die gleichzeitige oder sequentielle Abgabe der ersten und zweiten Zusammensetzungen an die Mundhöhle zu ermöglichen; und
(e) einen Satz von Anweisungen für den Anwender;
wobei die ersten und zweiten Reservoirs vom Gehäuse abgenommen und ersetzt werden können.

2. System nach Anspruch 1, wobei eine orale Behandlungszusammensetzung von 0,1 bis 40 % ein Reinigungsmittel umfasst, das ausgewählt ist aus Tensiden; Schleifmitteln und Mischungen davon.

3. System nach einem der vorstehenden Ansprüche, wobei eine orale Behandlungszusammensetzung von 0,01 bis 20 % ein Konditionierungsmittel umfasst, das ausgewählt ist aus tierischen Ölen, Mineralölen, Pflanzenölen und Silikonölen, Polymeren, die an den Oberflächen der Zähne oder den weichen Geweben des Mundes verbleiben; und Mischungen davon.

4. System nach einem der vorstehenden Ansprüche, wobei eine orale Behandlungszusammensetzung eine Fluoridionenquelle umfasst, die 100 bis 3000 ppm Fluoridionen bereitstellt.

5. System nach einem der vorstehenden Ansprüche, wobei die ersten und zweiten oralen Behandlungszusammensetzungen eine Viskosität von 10 bis 500 Pa.s, gemessen bei einer Scherrate von 0,1 s⁻¹ bei 25 °C, aufweisen.

6. System nach einem der vorstehenden Ansprüche, wobei das Steuermittel einen Mikroprozessor umfasst.

7. System nach Anspruch 6, wobei der Mikroprozessor programmierbar ist.

8. Kit, eine einheitliche Kartusche umfassend, die zur Verwendung in einem oralen Behandlungssystem nach einem der vorstehenden Ansprüche ausgelegt ist, wobei die Kartusche aufweist:
(a) erste und zweite Reservoirs, die eine erste bzw. eine zweite flüssige orale Behandlungszusammensetzung enthalten; und
(b) Medium, das einen Satz von Ausführungsinstruktionen zur Aktivierung einer Programmfunktion des oralen Behandlungssystems umfasst.

9. Kit nach Anspruch 8, wobei das Medium ein RFID-Tag ist.

## Revendications

1. Système de traitement buccal comprenant :
(a) un logement d'un seul tenant, qui peut être confortablement saisi dans la main d'un utilisateur, le logement contenant une cartouche comprenant :
(i) un premier réservoir contenant une première composition de traitement buccal liquide ;
(ii) un deuxième réservoir contenant une deuxième composition de traitement buccal liquide, qui est différente de la première composition de traitement buccal ;
(b) un applicateur fixé ou pouvant être fixé au logement pour appliquer les première et deuxième compositions de traitement buccal sur les dents ou les tissus mous buccaux ;
(c) une source d'alimentation électrique pour fournir de l'énergie pour pomper les compositions vers l'applicateur et / ou pour fournir de l'énergie à l'applicateur ;
(d) un moyen de contrôle pour permettre à l'utilisateur de libérer simultanément ou séquentiellement les première et deuxième compositions à la cavité buccale ; et
(e) un ensemble d'instructions pour l'utilisateur ;
dans lequel les premier et deuxième réservoirs sont amovibles de façon remplaçable du logement.

2. Système selon la revendication 1, dans lequel la composition de traitement buccal comprend de 0,1 à 40 % d'un agent de nettoyage choisi parmi des agents tensioactifs ; des abrasifs et leurs mélanges.

3. Système selon l'une quelconque des revendications précédentes, dans lequel la composition de traitement buccal comprend de 0,01 à 20 % d'un agent de conditionnement choisi parmi des huiles animales, des huiles minérales, des huiles végétales et des huiles de silicone, des polymères qui sont substantifs sur les surfaces des dents ou les tissus mous buccaux ; et leurs mélanges.

4. Système selon l'une quelconque des revendications précédentes, dans lequel la composition de traitement buccal comprend une source d'ion fluorure fournissant de 100 à 3000 ppm d'ion fluorure.

5. Système selon l'une quelconque des revendications précédentes, dans lequel les première et deuxième compositions de traitement buccal ont une viscosité allant de 10 à 500 Pa.s lorsqu'on mesure à une vitesse de cisaillement allant de 0,1 s⁻¹ à 25 °C.

6. Système selon l'une quelconque des revendications précédentes, dans lequel le moyen de contrôle comprend un microprocesseur.

7. Système selon la revendication 6, dans lequel le microprocesseur est programmable.

8. Trousse comprenant une cartouche d'un seul tenant adaptée pour une utilisation dans un système de traitement buccal selon l'une quelconque des revendications précédentes, la cartouche comprenant
(a) un premier et un deuxième réservoirs contenant respectivement une première et une deuxième compositions de traitement buccal liquides ; et
(b) un support comprenant un ensemble d'instructions exécutables pour activer une fonction de programme du système de traitement buccal.

9. Trousse selon la revendication 9, dans laquelle le support est une étiquette RFID.
